# EUROPÄISCHE PATENTSCHRIFT

(11) **EP 0 851 926 B1**
(45) Veröffentlichungstag und Bekanntmachung des Hinweises auf die Patenterteilung: **30.07.2003**
(21) Anmeldenummer: 96938915.4
(22) Anmeldetag: 15.08.1996
(51) Int. Cl.: C12N 15/23, C12N 15/70, C12N 7/01, C12N 1/21, C07K 1/22, C07K 14/57, A61K 38/21, G01N 33/50

(54) **VARIANTEN DES HUMANEN REKOMBINANTEN INTERFERON-GAMMA (RHU-IFN-GAMMA) MIT ERHÖHTER THERMISCHER STABILITÄT**
VARIANTS OF HUMAN RECOMBINANT INTERFERON-GAMMA (RHU-IFN-GAMMA) HAVING INCREASED HEAT-STABILITY
VARIANTES DE L'INTERFERON-GAMMA (RHU-IFN-GAMMA) HUMAIN RECOMBINE A STABILITE THERMIQUE RENFORCEE

(30) Priorität: 18.09.1995 DE 19535853
(43) Veröffentlichungstag der Anmeldung: 08.07.1998
(73) Patentinhaber: FRAUNHOFER-GESELLSCHAFT ZUR FÖRDERUNG DER ANGEWANDTEN FORSCHUNG E.V., 80636 München (DE)
(72) Erfinder: WASCHÜTZA, Gero, D-38536 Meinersen (DE); LI, Volkhart, D-51069 Köln (DE); OTTO, Bernd, D-30659 Hannover (DE)
(86) Internationale Anmeldenummer: DE9601556
(87) Internationale Veröffentlichungsnummer: WO97011179

(56) Entgegenhaltungen:
- EP-A- 0 170 917
- WO-A-92/08737
- DE-C- 4 036 855
- BIOCHEMICAL AND BIOPHYSICAL RESEARCH COMMUNICATIONS, Bd. 201, Nr. 3, 30.Juni 1994, Seiten 1401-1405, XP002027560 DENESYUK A.I. ET AL.: "Modelling of interhelical contacts in interferons-beta, -gamma and dimeric interleukin-5."
- SCIENCE, Bd. 252, 3.Mai 1991, Seiten 698-702, XP002027561 EALICK S.E.: "Three-dimensional structure of recombinant human interferon-gamma."
- JOURNAL OF INTERFERON RESEARCH, Bd. 12, 1992, Seiten 139-143, XP000647532 DAY C. ET AL. : "Engineered disulfide bond greatly increases specific activity of recombinant murine interferon-beta."
- PROTEIN ENGINEERING, Bd. 9, Nr. 10, Oktober 1996, Seiten 905-912, XP002027562 WASCHÜTZA G. ET AL.: "Engineered disulfide bonds in recombinant human interferon-gamma: the impact of the N-terminal helix A and the AB-loop on protein stability."

## Beschreibung

Die Erfindung betrifft Varianten des humanen rekombinanten Interferon-γ mit erhöhter thermischer Stabilität.

Interferon-γ eignet sich als antivirales, antiproliferatives, immunmodulatorisches Humantherapeutikum insbesondere für die Behandlung von Nierentumoren und chronischer Granulomatose.

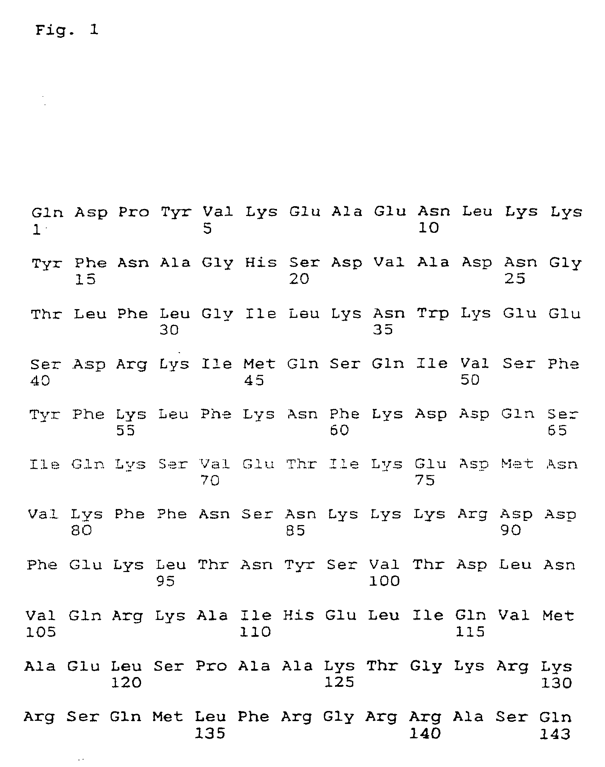

Interferane gehören, wie die Interleukine, zur Klasse der Cytokine und gliedern sich in unterschiedliche Klassen: Interferon-α, Interferon-β, Interferon-γ, Interferon-ω und Interferon-τ. Interferon-γ, ist ein Glycoprotein, dessen Aminosäuresequenz seit 1982 (Nucl. Acid Res. 10, S. 2487 ff (1982)) bekannt ist. In reifem Zustand besitzt das Interferon-γ 143 Aminosäuren und ein Molekulargewicht von 63 bis 73 Kilodalton. Die Aminosäuresequenz des Interferon-γ ist in Fig. 1 dargestellt. Es fällt auf, daß die Sequenz kein Cystein enthält.

Die Tertiär- und Quartärstruktur des nichtglycosylierten Proteins wurde 1991 aufgeklärt (Science 252,
S. 698 ff (1991)). Danach liegt Interferon-γ als Homodimer vor, wobei die Monomere gegensinnig so orientiert sind, daß das C-terminale Ende des einen Monomers sich in der Nähe des N-terminalen Endes des anderen Monomers befindet. Jedes dieser Monomere besitzt insgesamt sechs α-Helices.

Interferon-γ wird auch Immuninterferon genannt, da es unspezifische antivirale, antiproliferative und insbesondere immunmodulatorische Wirkungen besitzt. Seine Produktion in T-Helfer-Lymphozyten wird durch Mitogene und Antigene stimuliert. Die Wirkung des exprimierten Interferon-γ ist noch nicht genau geklärt, wird jedoch intensiv untersucht. Insbesondere führt Interferon-γ zur Aktivierung von Makrophagen und zur Synthese von Histokompatibilitätsantigenen der Klasse 2. In vitro wird die Aktivität des Interferon-γ gewöhnlich als Verringerung des virusinduzierten cytopatischen Effekts bestimmt, die sich aus der Behandlung mit Interferon-γ ergibt. Aufgrund seiner antigen-unspezifischen antiviralen, antiproliferativen und immunmodulatorischen Aktivität eignet es sich als Humantherapeutikum beispielsweise von Nierentumoren und chronischer Graunulomatose. Klinische Studien zur Therapie von verschiedenen Tumoren werden gegenwärtig durchgeführt. Große Hoffnungen werden auch in die Interferon-γ Therapie von Neurodermitis gesetzt. Weiterhin wird Interferon-γ im Forschungsbereich auch als Feinchemikalie beispielsweise zur Stimulation von Zellkulturen oder zur Bestimmung von Interferonspiegeln eingesetzt.

Seit 1982 können Varianten des humanen Interferon-γ in Bakterien wie Escherichia coli exprimiert werden (Nature 295, S. 503 ff (1982)). Das Monomer dieser rekombinanten Varianten des humanen Interferon-γ besitzt außer den 143 Aminosäuren des nativen humanen Monomers zusätzlich N-terminal Methionin als zusätzliche Aminosäure. Dieses rekombinante humane Interferon-γ (rhu-IFN-γ) ist nicht glycosyliert. Es kann in großen Mengen gewonnen werden und wurde daher sehr genau charakterisiert.

Auch das rekombinante humane Interferon-γ liegt als Dimer vor und besitzt normale biologische Aktivität.
Es ist säurelabil und temperaturempfindlich mit einem Schmelzpunkt von 52° C.

Von allen Interferonen ist die thermische Stabilität des humanen Interferon-γ am niedrigsten. Diese geringe thermische Stabilität des Interferon-γ erschwert seinen Einsatz als Humantherapeutikum.

Es wurden schon frühzeitig Versuche durchgeführt, um mit Hilfe von molekularbiologischen Verfahren Varianten mit verbesserten Eigenschaften des rekombinanten humanen Interferon-γ zu erzeugen. In der
EP 0 306870 A2 wurden Varianten des rekombinanten humanen Interferon-γ vorgestellt, deren Aktivität durch Abspaltung der C-terminalen 7-11 Aminosäuren signifikant erhöht wurde. Auch die WO 92-08737 gibt eine Variante des rekombinanten humanen Interferon-γ an (Interferon-γ C-10L), die eine erhöhte biologische Aktivität besitzt. Bei keiner dieser Varianten des rekombinanten humanen Interferons-γ wurde jedoch die thermische Stabilität signifikant erhöht. Die geringe thermische Stabilität erschwert daher auch für diese Proteine ihren Einsatz als Humantherapeutikum.

EP-A-0 170 917 beschreibt Mutanten des humanen Interferon γ mit erhöhter Hitzestabilität. In diesem Dokument wird an der Position 137 Cystein eingeführt, oder an den Positionen 1,3 und 135-138.

Ziel der vorliegenden Erfindung ist es, weitere Varianten des rekombinanten humanen Interferon-γ zur Verfügung zu stellen, deren thermische Stabilität erhöht ist, wobei die biologische Aktivität ähnlich, vorzugsweise gleich oder gar besser als die des unveränderten rekombinanten humanen Interferon-γ ist. Weiterhin ist es Ziel dieser Erfindung, Gensequenzen, Vektoren mit Gensequenzen und Mikroorganismen mit Gensequenzen, die zur Herstellung des Expressionssystems und der erfindungsgemäßen Varianten des Interferon-γ verwendet werden können, anzugeben.

Die Aufgabe wird dadurch gelöst, daß DNA-Sequenzen und rekombinante Vektoren mit derartigen DNA-Sequenzen, beispielsweise Plasmide und Bakteriophagen, sowie Mikroorganismen, die derartige DNA-Seguenzen enthalten, angegeben werden, die für Polypeptide kodieren, die sich gegenüber rekombinantem humanem Interferon-γ durch erhöhte thermische Stabilität auszeichnen. Weiterhin werden Verfahren angegeben, durch die derartige DNA-Sequenzen, rekombinante Vektoren und Mikroorganismen hergestellt werden können. Mit Hilfe der erfindvingsgemäßen Oligonukleotide und
entsprechender Restrikionsendonukleasen können ausgehend von einem an sich bekannten Plasmid, das das Gen für rekombinantes humanes Interferon-γ enthält, rekombinante Vektoren wie Plasmide oder Bakteriophagen sowie Mikroorganismen hergestellt werden, die eine DNA-Sequenz enthalten, die für die erfindungsgemäßen Polypeptide kodieren.

Weiterhin wird ein Herstellungsverfahren für die erfindungsgemäßen Proteine angegeben, durch das mit Hilfe der erfindungsgemäßen Mikroorganismen die erfindungsgemäßen Proteine gewonnen, konzentriert und aufgereinigt werden können, sowie Verwendungen der erfindungsgemäßen Proteine als Humantherapeutikum oder/und Feinchemikalie.

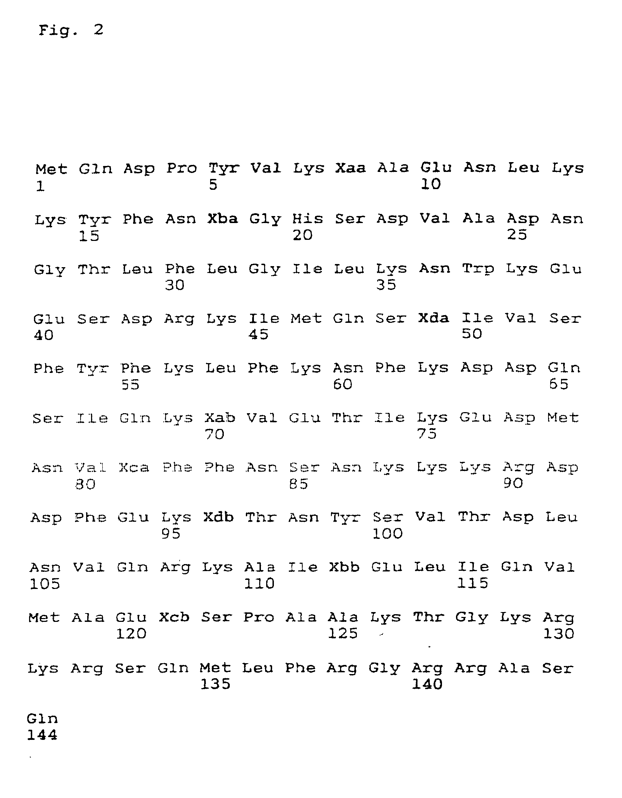

Die erfindungsgemäßen Polypeptide bestehen aus einer Sequenz von 144 Aminosäuren, die sich aus der Sequenz der 143 Aminosäuren des rekombinanten humanen Interferon-γ und einem zusätzlichen N-terminalen Methionin zusammensetzt. Außerdem unterscheidet sich das erfindungsgemäße Polypeptid von dem Monomer des rekombinanten humanen Interferon-γ dadurch, daß mindestens ein Paar Aminosäuren aus vier vorbestimmten Aminosäurepaaren gegen Cystein ausgetauscht ist. Diese vier Aminosäurepaare sind Glu8 und Ser70, Ala18 und His112, Lys81 und Leu121 sowie Gln49 und Leu96. Die vollständige Aminosäuresequenz ist für das rekombinante humane Interferon-γ in Fig. 1 und für das erfindungsgemäße Polypeptid in Fig. 2 angegeben. Dabei bedeuten Xaa Glu8, Xab Ser70, Xba Ala18, Xbb His112, Xca Lys81, Xcb Leu121, Xda Gln49 sowie Xdb Leu96, sofern diese Aminosäuren in den erfindungsgemäßen Polypeptiden nicht für Cystein stehen.

Überraschenderweise stellte sich heraus, daß die erfindungsgemäßen Proteine eine höhere thermische Stabilität verglichen mit dem rekombinanten humanen Interferon-γ besitzen.

Die erfindungsgemäßen Proteine sind wahrscheinlich ebenso wie das rekombinante humane Interferon-γ Homodimere, deren Monomere so orientiert sind, daß das N-terminale Ende des einen Monomers sich in der Nähe des C-terminalen Endes des anderen Monomers befindet. Möglicherweise führt der paarweise Austausch der Aminosäuren zu zusätzlichen intermonomeren und intramonomeren Disulfidbrücken. So führt der Austausch von Ala18 und His112 sowie der Austausch von Lys80 und Leu120 wahrscheinlich zu intermonomeren Disulfidbrücken, und der Austausch von Glu8 und Ser70 zu einer intramonomeren Disulfidbrücke, während der Austausch von Glu49 und Leu96 wahrscheinlich zu keiner zusätzlichen kovalenten Bindung führt. Offensichtlich kann. aus der Einführung von Cysteinpaaren in die Aminosäuresequenz des Polypeptidmonomers nicht unmittelbar auf die Ausbildung von Disulfidbrücken geschlossen werden.

Aufgrund der gegenüber dem herkömmlichen rekombinanten humanen Interferon-γ verbesserten Eigenschaften der Proteine und Polypeptide nach den Ansprüche 1, 2 und 3 eignen sich diese Proteine besonders für verschiedene Anwendungsformen als Humantherapeutikum. Sie sind insbesondere in denjenigen Bereichen vorteilhaft anwendbar, bei denen auch bisher schon humanes rekombinantes Interferon-γ als Arzneimittel verwendet wurde, wie zum Beispiel Nierentumoren oder chronischer Granulomatose. Weiterhin ist zu erwarten, daß die erfindungsgemäßen Proteine auch bei allen zukünftigen Indikationen verwendet werden können, bei denen herkömmliches rekombinantes humanes Interferon-γ als Arzneimittel Verwendung finden wird.

Die erfindungsgemäßen Varianten des rekombinanten humanen Interferon-γ eignen sich auch für die Verwendung als Feinchemikalie beispielsweise für in vitro-Versuche, zur Bestimmung von Interferonspiegeln sowie zur Stimulation von Zellkulturen. Aufgrund ihrer höheren thermischen Stabilität und damit besseren Handhabbarkeit und aufgrund ihrer antiviralen, antiproliferativen und immunodulatorischen biologischen Aktivität können sie in allen Bereichen, in denen herkömmliches rekombinantes humanes Interferon-γ verwendet wird, dieses ersetzen.

Weitere vorteilhafte Ausführungsformen werden in den abhängigen Ansprüchen geschildert.

Eine Verkürzung des C-terminalen Endes des Polypeptid-Monomers um eine bis zehn Aminosäuren führt zu einer Verbesserung der biologischen Aktivität. Dadurch wird erreicht, daß sowohl die thermische Stabilität der erfindungsgemäßen Varianten des rekombinanten humanen Interferon-γ als auch seine biologische Aktivität gegenüber dem rekombinanten humanen Interferon-γ verbessert wird.

Im folgenden wird die Herstellung der neuen rekombinanten Vektoren und Mikroorganismen beschrieben.

Zur Herstellung eines rekombinanten Mikroorganismus, der eine Gensequenz enthält, die für eines der erfindungsgemäßen Polypeptide kodiert, wird die Sequenz des humanen rekombinanten Interferon-γ mit Hilfe von Restriktionsendonukleasen aus einem Vektor, der diese Sequenz enthält herausgeschnitten und in die DNA eines Phagen kloniert. Die Phagen-DNA wird anschließend mit Hilfe geeigneter Oligonukleotide mutiert, so daß sie für eines der erfindungsgemäße Polypeptide kodiert. Der genetisch veränderte Phage wird anschließend vermehrt und die veränderte Sequenz für humanes rekombinantes Interferon-γ aus der DNA des Phagen mithilfe derselben Restriktionsendonukleasen ausgeschnitten und in das Plasmid zurückkloniert. Mit Hilfe dieses Plasmids wird nun ein Mikroorganismus transfiziert, welcher anschließend das neue für das erfindungsgemäße Polypeptid codierende Gen exprimieren kann.

Dieses Verfahren soll nun am Beispiel eines transfizierten Bakteriums Escherichia coli K12 Stamm JM 105 dargestellt werden.

Als Ausgangsmaterial wurde das bekannte Plasmid pKK-233-2/IFN-γ verwendet. Dieses Plasmid wurde mit den Restriktionsendonukleasen EcoRI und HindIII verdaut. Dieses Plasmid besitzt zwischen den beiden Schnittstellen für EcoRI und HindIII das Gen für humanes rekombinantes Interferon-γ und einen starken IPTG-induzierbaren trc-Promotor. Dieser DNA-Abschnitt mit einer Länge von 751 Basenpaaren wurde anschließend ebenfalls mit EcoRI und HindIII in den ebenfalls bekannten Bakteriophagen M13mp18 kloniert. Anschließend wurde die DNA des Bakteriophagen mit an sich bekannten Methoden und geeigneten Oligonukleotiden so mutiert, daß das Gen für Interferon-γ für eine der erfindungsgemäßen Varianten kodiert. Dieser neue Bakteriophage M13mp18/IFN-γ wurde in dem Bakterium Escherichia coli K12 Stamm TG1 vermehrt. Aus der cDNA des vermehrten Phagen wurde mithilfe derselben Restriktionsendonukleasen EcoRI und HindIII der nunmehr veränderte Sequenzabschnitt, der das Gen für das veränderte rhu-IFN-γ enthielt, ausgeschnitten und in das Plasmid pKK233-2 zurückklonisrt. Dieses nunmehr ebenfalls veränderte Plasmid pKK233-2/IFN-γ wurde nun verwendet, um das Bakterium Escherichia coli K12 Stamm JM205 zu transfizieren.

Die verwendeten Oligonukleotide besitzen eine Länge von 29 Basenpaaren und eine Sequenz, die komplementär ist zu der einzelsträngigen DNA-Sequenz der entsprechenden Bakteriophagen-DNA, die etwa mittig die zu mutierenden Basenpaare enthält. Als komplementäres Codon für die einzuführende Aminosäure Cystein wurde sowohl ACA als auch GCA verwendet. Diese Oligonukleotidprimer wurden mit einem handelsüblichen DNA-Synthesizer hergestellt. Es wurde kein Unterschied in der Mutationsrate zwischen den beiden Codons für Cystein gefunden.

Zur Herstellung der erfindungsgemäßen Proteine wird der transformierte rekombinante Mikroorganismus kultiviert und anschließend aus der Kultur das erfindungsgemäße Protein abgetrennt, aufgereinigt und konzentriert.

Das Herstellungsverfahren für die erfindungsgemäßen Proteine wird nun beispielhaft anhand der oben erwähnten transfektierten Escherichia coli K12 Stamm JM105 dargestellt.

Die transfektierten Bakterien enthalten die Gensequenz für das veränderte Interferon-γ und einen durch IPTG induzierbaren trc-Promotor. Die Bakterien werden in Kulturen genommen und die Expression des mutierten Interferon-γ wird durch Zugabe von IPTG induziert. Das exprimierte veränderte Interferon-γ wird von den Bakterienzellen in sogenannten "Inclusion bodies" eingelagert. Für die Reinigung des exprimierten, veränderten Interferon-γ werden die Bakterienzellen nach erfolgreicher Expression aufgebrochen und die "Inclusion bodies" durch mehrfaches Waschen von löslichen bakteriellen Proteinen befreit. Das Aufbrechen wird bevorzugt mechanisch, beispielsweise durch Ultraschall, vorgenommen. Das erfindungsgemäße Protein wird durch einen Denaturierungsschritt mit Guanidiniumhydrochlorid in Lösung gebracht und abgetrennt. Anschließend wird das erfindungsgemäße Protein durch Verdünnen in einem Phosphatpuffer renaturiert und in seine biologisch aktive Form gefaltet. Das dadurch zu mehr als 90 % saubere Interferon-γ wird mit einer Rate von bis zu 30 % des totalen Proteingehalts der E.coli-Kultur gewonnen. Es kann anschließend durch eine Affinitätschromatographie auf einer Säule oder in einem chargenweisen verfahren konzentriert und weiter gereinigt werden und erreicht nach einem weiteren Filtrationsschritt, beispielsweise einer HPLC-Gelfiltration, einen Reinheitsgrad von mehr als 95 %.

Unter einem chargenweisen Verfahren im Sinne dieser Erfindung, auch Batch-Verfahren genannt, wird folgendes verstanden: das chromatographische Material wird gleichmäßig so in eine Lösung des erfindungsgemäßen Proteins eingerührt, daß das veränderte Interferon-γ gleichmäßig verteilt an das chromatographische Material bindet und die Interferon-γ-Proteinkonzentration nicht mehr als ca. 2 mg/ml gepacktes chromatographisches Material beträgt. Das mit Interferon-γ beladene chromatographische Material wird im Batch mit Phosphatpuffer gewaschen und das veränderte Interferon-γ dann im Batch mit Kochsalzlösung in Phosphatpuffer eluiert. Als chromatographisches Material können hier zum Beispiel SP-Sepharose oder Affi-Gel-Blue angewendet werden.

Die Figuren zeigen in
- Fig. 1: die Aminosäuresequenz des humanen rekombinanten Interferon-γ;
- Fig. 2: die Aminosäuresequenz der Varianten des rekombinanten humanen Interferon-γ nach Anspruch 1;

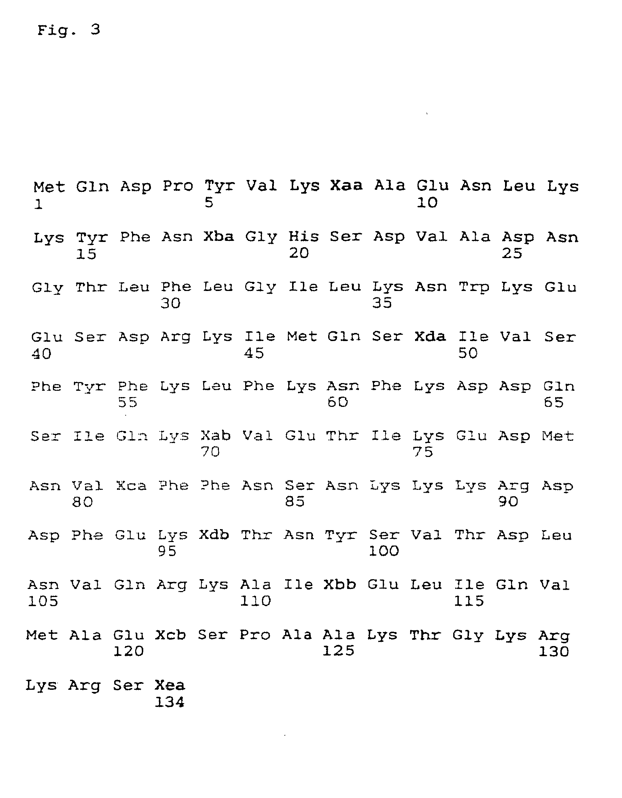

- Fig. 3: die Aminosäuresequenz der Varianten des rekombinanten humanen Interferon-γ entsprechend Anspruch 2; und

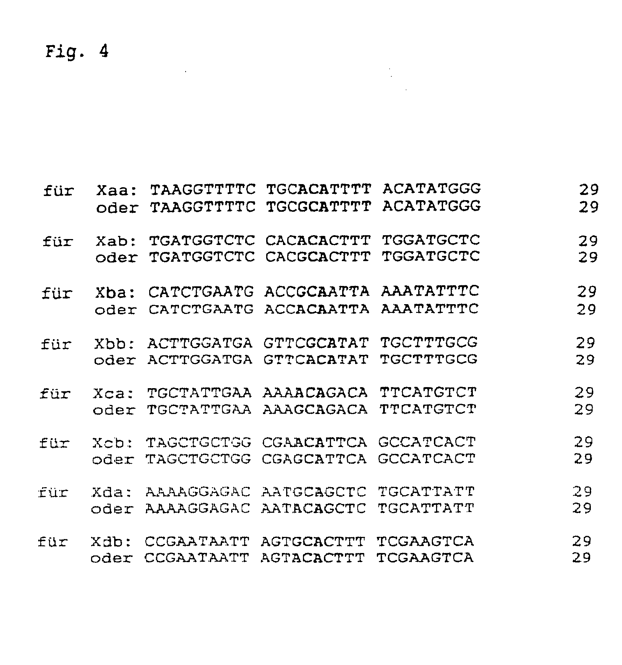

- Fig. 4: die Basensequenz der Oligonukleotide, die zur Herstellung der rekombinanten DNA, die für die erfindungsgemäßen Proteine kodiert, verwendet wurden.

Im folgenden werden einige beispielhafte Ausführungen der Erfindung erläutert.

### Beispiel 1

Es wurden die Aminosäuren Glu8 und Ser70 nach dem folgenden Verfahren ausgetauscht und ein entsprechendes verändertes Interferon-γ hergestellt.

Das Interferon-γ-Gen des Plasmids pKK-233-2/IFN-γ wurde in den Phagen M13mp18 umkloniert. Dazu wurde eine Plasmidpräparation mit den Restriktionsendonukleasen EcoRI und HindIII verdaut. Durch diesen Verdau wurde der Vektor in zwei Fragmente mit den Längen 751 Basenpaare und 4305 Basenpaare gespalten. Beide Fragmente wurden über ein 1%iges Agarosegel voneinander getrennt und die entsprechende 751 Basenpaare große Bande aus dem Gel ausgeschnitten und isoliert. Dieses Fragment enthielt außer dem kompletten Interferon-γ-Gen den trc-Promotor und weitere Vektorenanteile am 5'-Ende.

Eine 150 ml Kultur von Escherichia coli TG1 wurde mit dem Phagen M13mp18 infiziert. Nach einer fünfstündigen Inkubation wurden die Zellen abzentrifugiert, einmal resuspendiert und erneut pelletiert. Die doppelsträngige, zirkuläre Phagen-DNA wurde mit dem Quiagen™-Plasmidisolierungskit (Firma Diagen) aus den Zellen aufgereinigt. Die RF-DNA des Phagen M13mp18 wurde ebenfalls mit den Restriktionsenzymen EcoRI und HindIII verdaut und über ein 1 %iges Agarosegel gereinigt. Anschließend wurde das 751 bp lange, isolierte EcoRI/HindIII-Insert aus dem Vektor pKK-233-2/IFN-γ in den Phagen M13mp18 kloniert.

Um Expressionsvektoren für die neue Variante des Interferon-γ herzustellen, wurden Mutationen der DNA-Sequenz an definierten Stellen durchgeführt. Das Verfahren basiert auf der Hybridisierung eines Oligonukleotides mit der gewünschten Zielsequenz auf einzelsträngiger DNA (ssDNA) des zu verändernden Phagen M13. Zur Erzeugung der beiden Punktmutationen von Glu8 und Ser70 zu Cystein wurde folgende Oligonukleotide verwendet:
für die Mutation von Glu8 zu Cys 
TAAGGTTTTC TGCACATTTT ACATATGGG 29
oder
TAAGGTTTTC TGCGCATTTT ACATATGGG 29
 und für die Mutation von Ser70 zu Cys

TGATGGTCTC CACACACTTT TGGATGCTC 29
oder
TGATGGTCTC CACGCACTTT TGGATGCTC 29

Die Oliglonukleotide wurden mit Hilfe eines handelsüblichen Synthesizers hergestellt. Die beiden Codons für cystein sind TGT und TGC. Die entsprechende komplementäre Busenfolge im Nukleotidstrang ist GCA oder ACA. Beide Codons wurden verwendet (hervorgehoben). Als Länge aller Oligonukleotid-Primer wurden 29 Basenpaare gewählt. Für die Mutagenese mußten alle Oligonukleotide an ihrem 5'-Ende mit T4-Polynukleotidkinase phosphoryliert werden.

Unabhängig von der Sequenz der Oligonukleotide wurde die Hybridisierung bei 80° C im Wasserbad durchgeführt. Nach Durchführung der in vitro-Mutagenese wurde die Transformation der doppelsträngigen RF-Form der Phagen-DNA analog zu einem normalen Plasmid mit der CaCl₂-Methode durchgeführt. Zellen von E. coli wurden mit der rekombinanten Phagen-RF-DNA, IPTG und X-Gal zusammengegeben und ausplattiert. Nach Inkubation über Nacht bei 37° C wurden von jeder Mutagenese-Reaktion jeweils 14 Phagen-Klone gepickt und erneut zusammen mit E.coli für fünf Stunden bei 37° C inkubiert.

Die Sequenzkontrolle der mutierten cDNA der Bakteriophagen, die für die neue Variante des rekombinanten humanen Interferon-γ codieren soll, erfolgte durch Didesoxy-Sequenzierung mit dem T7-Sequenzierungs-Kit von Pharmacia. Damit wurden die gewünschten Mutationen bestätigt.

Anschließend an die Mutagenese der Phagen-DNA wurde die 751 bp lange Sequenz, die für das veränderte rhu-IFN-γ kodierte, mithilfe derselben Restriktionsendonukleasen, EcoRI und HindIII, aus der Phagen-DNA ausgeschnitten und in den Phagen pKK233-2 zurückkloniert. Mit diesem Plasmid wurde dann das Bakterium Escherichia coli K12 Stamm JM105 transfiziert.

Die Expression des veränderten Interferon-γ wurde nach den oben beschriebenen Methoden durchgeführt. Die biologisch, antivirale Aktivität des veränderten Interferon-γ wurde durch die Verringerung des virusinduzierten cytopathischen Effekts bestimmt, die sich nach der Behandlung mit dem veränderten Interferon-γ ergab. Als Zellinie wurden humane Lungenfibroblasten A549 und als Virus der Enzephalomyokarditis-Virus verwendet.

Die Analyse des erhaltenen veränderten Interferon-γ ergab, daß seine biologische Aktivität im Vergleich zum rekombinanten humanen Interferon-γ unverändert war. Im Gegensatz zum rekombinanten humanen Interferon-γ mit einer Schmelztemperatur von 52,2° C war die Schmelztemperatur der neuen Variante mit 68,5° C erheblich erhöht. Bei gleicher biologischer Aktivität ergab sich folglich für die neue Variante des Interferon-γ eine erheblich verbesserte thermische Stabilität gegenüber dem unveränderten Interferon-γ.

### Beispiel 2

Es wurde eine Variante des rekombinanten humanen Interferon-γ hergestellt, bei der die beiden Aminosäuren Ala18 und His112 gegen Cystein ausgetauscht wurden. Die Erzeugung der entsprechenden DNA, Phagen-DNA, Plasmid-DNA und der entsprechenden Expressionsorganismen wurde wie in Beispiel 1 durchgeführt. Für die Hybridisierung der ssDNA wurden folgende Oligonukleotide verwendet:
für die Mutation von Ala18 zu Cys 
CATCTGAATG ACCGCAATTA AAATATTTC 29
oder
CATCTGAATG ACCACAATTA AAATATTTC 29
 und für die Mutation von His112 zu Cys

ACTTGGATGA GTTCGCATAT TGCTTTGCG 29
oder
ACTTGGATGA GTTCACATAT TGCTTTGCG 29

Auch bei dieser Variante des rekombinanten humanen Interferon-γ ergab sich eine erhöhte thermische Stabilität mit einem Schmelzpunkt des Interferon-γ von 78,6° C bei gleichzeitigen Nachweis antiviraler Aktivität der neuen Variante.

### Beispiel 3

Es wurde eine variante des rekombinanten, humanen Interferon-γ hergestellt, bei der die beiden Aminosäuren Lys81 und Leu121 gegen Cystein ausgetauscht wurden. Die Herstellung der entsprechenden DNA, Plasmid-DNA, Phagen-DNA und des entsprechenden Expressionsmikroorganismus erfolgte wie in Beispiel 1 beschrieben. Zur Herstellung der Variante wurden folgende Oligonukleotide verwendet:
für die Mutation von Lys81 zu Cys 
TGCTATTGAA AAAACAGACA TTCATGTCT 29
oder
TGCTATTGAA AAAGCAGACA TTCATGTCT 29
 und für die Mutation von Leu121 zu Cys

TAGCTGCTGG CGAACATTCA GCCATCACT 29
oder
TAGCTGCTGG CGAGCATTCA GCCATCACT 29

Diese Variante des rekombinanten humanen Interferon-γ zeigte eine dem unveränderten rekombinanten Interferon-γ ähnliche biologische, antivirale Aktivität bei verbesserter thermischer Stabilität.

### Beispiel 4

Es wurde eine Variante des rekombinanten humanen Interferon-γ hergestellt, bei der die beiden Aminosäuren Gln49 und Leu96 gegen Cystein ausgetauscht wurden. Die Herstellung der entsprechenden DNA, Plasmid-DNA, Phagen-DNA und des entsprechenden Expressionsmikroorganismus wurde ebenfalls wie in Beispiel 1 geschildert durchgeführt. Zur Herstellung dieser Variante des Interferon-γ wurden die folgenden Oligonukleotide verwendet:
für die Mutation von Gln49 zu Cys 
AAAAGGAGAC AATGCAGCTC TGCATTATT 29
oder
AAAAGGAGAC AATACAGCTC TGCATTATT 29
 und für die Mutation von Leu96 zu Cys

CCGAATAATT AGTGCACTTT TCGAAGTCA 29
oder
CCGAATAATT AGTACACTTT TCGAAGTCA 29

Auch für diese Variante des Interferon-γ ergaben sich die in den vorhergehenden Beispielen geschilderten Vorteile. Dabei ist auch diese Variante des Interferon-γ mit einer antiviralen Aktivität, die ca. 30 % des unveränderten rekombinanten humanen Interferon-γ entspricht, für die erfindungsgemäßen Verwendungen als Therapeutikum oder als Feinchemikalie, wie zum Beispiel zur Stimulation von Zellkulturen, geeignet.

### Beispiel 5

Es wurde eine Variante des in Beispiel 1 gezeigten veränderten rekombinanten humanen Interferon-γ, bei dem die Aminosäuren Glu8 und Ser70 gegen Cystein ausgetauscht wurden, hergestellt, wobei zusätzlich in ansonsten bekannter Weise die Aminosäuresequenz des monomeren Polypeptids C-terminal um 10 Aminosäuren verkürzt wurde.

Bei erhöhter thermischer Stabilität zeigt diese Variante des rekombinanten humanen Interferon-γ eine Aktivität, die um das 1- bis 4fache höher als die antivirale Aktivität des unveränderten rekombinanten humanen Interferon-γ liegt. Damit wird eine Variante des Interferon-γ vorgestellt, die neben erhöhter thermischer Stabilität zugleich erhöhte biologische Aktivität zeigt.

## Patentansprüche

1. Polypeptid mit folgender Aminosäuresequenz: 
Met Gln Asp Pro Tyr Val Lys Xaa Ala Glu Asn Leu
1               5                   10

Lys Lys Tyr Phe Asn Xba Gly His Ser Asp Val Ala
        15                  20

Asp Asn Gly Thr Leu Phe Leu Gly Ile Leu Lys Asn
25                  30                  35

Trp Lys Glu Glu Ser Asp Arg Lys Ile Met Gln Ser
            40                  45

Xda Ile Val Ser Phe Tyr Phe Lys Leu Phe Lys Asn
    50                  55                  60

Phe Lys Asp Asp Gln Ser Ile Gln Lys Xab Val Glu
                65                  70

Thr Ile Lys Glu Asp Met Asn Val Xca Phe Phe Asn
        75                  80

Ser Asn Lys Lys Lys Arg Asp Asp Phe Glu Lys Xdb
85                  90                  95

Thr Asn Tyr Ser Val Thr Asp Leu Asn Val Gln Arg
            100                 105

Lys Ala Ile Xbb Glu Leu Ile Gln Val Met Ala Glu
    110                 115                 120

Xcb Ser Pro Ala Ala Lys Thr Gly Lys Arg Lys Arg
                125                 130

Ser Gln Met Leu Phe Arg Gly Arg Arg Ala Ser Gln
        135                 140             144
 wobei Xaa und Xab, Xba und Xbb, Xca und Xcb bzw. Xda und Xdb jeweils Paare von Aminosäuren bezeichnen und die Aminosäuren mindestens eines dieser Paare paarweise Cystein sind und wobei für die Aminosäuren, die nicht Cystein sind, gilt:
Xaa = Glu, Xab = Ser
Xba = Ala, Xbb = His
Xca = Lys, Xcb = Leu
Xda = Gln, Xdb = Leu,
deren C-Terminal um 1 bis 10 Aminosäuren verkürzte Varianten und deren Homodimere.

2. DNA-Sequenz,
**dadurch gekennzeichnet, daß** sie für ein Polypeptid nach Anspruch 1 kodiert.

3. Rekombinanter Vektor,
**dadurch gekennzeichnet, daß** er DNA enthält, die für ein Polypeptid nach Anspruch 1 kodiert.

4. Rekombinanter Vektor nach Anspruch 3,
**dadurch gekennzeichnet, daß** er ein Plasmid ist.

5. Rekombinanter Vektor nach Anspruch 4,
**dadurch gekennzeichnet, daß** er aus dem Plasmid pKK233-2/IFN-γ besteht, wobei seine Gen-Sequenz für ein Polypeptid nach Anspruch 1 kodiert.

6. Rekombinanter Vektor nach Anspruch 3,
**dadurch gekennzeichnet, daß** er aus einem Bakteriophagen besteht, der DNA enthält, die für ein Polypeptid nach Anspruch 1 kodiert.

7. Rekombinanter Vektor nach Anspruch 6,
**dadurch gekennzeichnet, daß** er aus dem Bakteriophagen M13mp19 besteht, in den eine Gensequenz kloniert wurde, die für ein Polypeptid nach Anspruch 1 kodiert.

8. Rekombinanter Mikroorganismus,
**dadurch gekennzeichnet, daß** er mit einem Vektor nach einem der Ansprüche 3 bis 7 transformiert wurde.

9. Mikroorganismus nach Anspruch 8,
**dadurch gekennzeichnet, daß** er ein Bakterium ist.

10. Mikroorganismus nach Anspruch 9,
**dadurch gekennzeichnet, daß** das Bakterium Escherichia coli K12 Stamm JM105 ist.

11. Verfahren zur Herstellung eines rekombinanten Bakteriophagen nach einem der Ansprüche 6 oder 7,
**dadurch gekennzeichnet, daß** das Plasmid pKK233-2/IFN-γ mit Restriktionsendonukleasen verdaut wird und anschließend der DNA-Abschnitt, der die für Interferon-γ kodierende Sequenz enthält, in ansonsten bekannter Weise in die DNA des Bakteriophagen M13mp18 kloniert und dann die rekombinante DNA des Bakteriophagen mit Hilfe von Oligonukleotiden in ebenfalls ansonsten bekannter Weise so modifiziert wird, daß sie für ein Polypeptid nach Anspruch 1 kodiert.

12. Verfahren nach Anspruch 11,
**dadurch gekennzeichnet, daß** als Restriktionsendonukleasen EcoRI und HindIII verwendet werden.

13. Verfahren zur Herstellung eines rekombinanten Bakteriums nach einem der Ansprüche 6 oder 7,
**dadurch gekennzeichnet, daß** zur Transfektion des Mikroorganismus ein Plasmid nach einem der Ansprüche 4 oder 5 verwendet wird.

14. Verfahren zur Herstellung eines Polypeptids nach Anspruch 1,
**dadurch gekennzeichnet, daß** ein transformiertes Bakterium nach einem der Ansprüche 8 bis 10 in Kultur gezüchtet wird, diese Zellen aufgebrochen werden und das Protein in ansonsten bekannter Weise abgetrennt und aufgereinigt wird.

15. Verfahren nach Anspruch 14,
**dadurch gekennzeichnet, daß** die Bakterienzellen mechanisch, z.B. mit Ultraschall, aufgebrochen werden.

16. Verfahren nach einem der Ansprüche 14 oder 15,
**dadurch gekennzeichnet, daß** die Abtrennung des Proteins erfolgt, indem die Zellvesikel, die das Protein enthalten, mindestens einmal gewaschen werden und das Protein anschließend mit Guanidiniumhydrochlorid in Lösung gebracht und nach Abtrennung durch Verdünnen renaturiert wird.

17. Verfahren nach mindestens einem der Ansprüche 14 bis 16,
**dadurch gekennzeichnet, daß** das Protein durch eine Affinitätschromatographie auf einer Säule oder im Batch-Verfahren isoliert und anschließend durch eine Gelfiltration hochgereinigt wird.

18. Verfahren nach mindestens einem der Ansprüche 14 bis 17,
**dadurch gekennzeichnet, daß** beim Batch-Verfahren das Material der Affinitätschromatographie in gleichmäßigen Kontakt mit der Proteinlösung gebracht wird, um eine gleichmäßige Belegung des Harzes zu erreichen, und daß anschließend im Batch gewaschen und eluiert wird.

19. Verfahren nach mindestens einem der Ansprüche 17 oder 18,
**dadurch gekennzeichnet, daß** für die Affinitätschromatographie übliche Materialien, wie z.B. SP-Sepharose oder Affi-Gel-Blue, verwendet werden.

20. Verwendung eines Polypeptids nach Anspruch 1 zur Herstellung eines Arzneimittels bei Nierentumoren, chronischer Granulomatose oder Neurodermitis.

21. Verwendung eines Polypeptids nach Anspruch 1 als Feinchemikalie für in-vitro-Versuche, für in vitro Interferonspiegel-Messungen und/oder zur Stimulation von Zellkulturen.

## Claims

1. Polypeptide having the following amino acid sequence: 
Met Gln Asp Pro Tyr Val Lys Xaa Ala Glu Asn Leu
1               5                   10

Lys Lys Tyr Phe Asn Xba Gly His Ser Asp Val Ala
        15                  20

Asp Asn Gly Thr Leu Phe Leu Gly Ile Leu Lys Asn
25                  30                  35

Trp Lys Glu Glu Ser Asp Arg Lys Ile Met Gln Ser
            40                  45

Xda Ile Val Ser Phe Tyr Phe Lys Leu Phe Lys Asn
    50                  55                  60

Phe Lys Asp Asp Gln Ser Ile Gln Lys Xab Val Glu
                65                  70

Thr Ile Lys Glu Asp Met Asn Val Xca Phe Phe Asn
        75                  80

Ser Asn Lys Lys Lys Arg Asp Asp Phe Glu Lys Xdb
85                  90                  95

Thr Asn Tyr Ser Val Thr Asp Leu Asn Val Gln Arg
            100                 105

Lys Ala Ile Xbb Glu Leu Ile Gln Val Met Ala Glu
    110                 115                 120
 
Xcb Ser Pro Ala Ala Lys Thr Gly Lys Arg Lys Arg
125 130

Ser Gln Met Leu Phe Arg Gly Arg Arg Ala Ser Gln
135 140 144
 wherein Xaa and Xab, Xba and Xbb, Xca and Xcb or Xda and Xdb in each case designate pairs of amino acids and the amino acids of at least one of these pairs are cysteine in pairs and wherein the following applies for the amino acids which are not cysteine:
Xaa = Glu, Xab = Ser
Xab = Ala, Xbb = His
Xca = Lys, Xcb = Leu
Xda = Gln, Xdb = Leu,
the variants of same in which the C-terminal end is shortened by 1 to 10 amino acids, and their homodimers.

2. DNA sequence, **characterised in that** it codes for a polypeptide according to claim 1.

3. Recombinant vector, **characterised in that** it contains DNA which codes for a polypeptide according to claim 1.

4. Recombinant vector according to claim 3, **characterised in that** it is a plasmid.

5. Recombinant vector according to claim 4, **characterised in that** it comprises the plasmid pKK233-2/IFN-g, its gene sequence coding for a polypeptide according to claim 1.

6. Recombinant vector according to claim 3, **characterised in that** it comprises a bacteriophage containing DNA which codes for a polypeptide according to claim 1.

7. Recombinant vector according to claim 6, **characterised in that** it comprises the bacteriophage M13mp19 into which a gene sequence has been cloned which codes for a polypeptide according to claim 1.

8. Recombinant micro-organism, **characterised in that** it has been transformed with a vector according to one of claims 3 to 7.

9. Micro-organism according to claim 8, **characterised in that** it is a bacterium.

10. Micro-organism according to claim 9, **characterised in that** the bacterium is Escherichia coli K12, strain JM105.

11. Method of producing a recombinant bacteriophage according to one of claims 6 or 7, **characterised in that** the plasmid pKK233-2/IFN-g is digested with restriction endonucleases and then the DNA segment, which contains the sequence coding for interferon-g, is cloned in otherwise known manner into the DNA of the bacteriophage M13mp18, and then the recombinant DNA of the bacteriophage is so modified with the aid of oligonucleotides in an also otherwise known manner, that it codes for a polypeptide according to claim 1.

12. Method according to claim 11, **characterised in that** EcoRI and HindIII are used as restriction endonucleases.

13. Method of producing a recombinant bacterium according to one of claims 6 or 7, **characterised in that** a plasmid according to one of claims 4 or 5 is used for the transfection of the micro-organism.

14. Method of producing a polypeptide according to claim 1, **characterised in that** a transformed bacterium according to one of claims 8 to 10 is grown in culture, these cells are broken up and the protein is separated and cleaned up in otherwise known manner.

15. Method according to claim 14, **characterised in that** the bacterium cells are broken up mechanically, e.g. with ultrasound.

16. Method according to one of claims 14 or 15, **characterised in that** the protein is separated by the cell vesicles, which contain the protein, being washed at least once and the protein being then made into a solution with guanidium hydrochloride and being renaturated after separation by dilution.

17. Method according to at least one of claims 14 to 16, **characterised in that** the protein is isolated by affinity chromatography on a column or in a batch process and then highly purified by gel filtration.

18. Method according to at least one of claims 14 to 17, **characterised in that** in the batch process, the material of the affinity chromatography is brought into uniform contact with the protein solution, in order to achieve uniform occupancy of the resin, and **in that** there is then washing and elution in the batch.

19. Method according to at least one of claims 17 or 18, **characterised in that** usual materials, such as SP Sepharose or Affi-Gel Blue, are used for the affinity chromatography.

20. Use of a polypeptide according to claim 1 to produce a medication for kidney tumours, chronic granulomatosis or neurodermatitis.

21. Use of a polypeptide according to claim 1 as a fine chemical for in-vitro experiments, for in-vitro interferon level measurements and/or for the stimulation of cell cultures.

## Revendications

1. Polypeptide ayant la séquence d'aminoacides suivante : 
Met Gln Asp Pro Tyr Val Lys Xaa Ala Glu Asn Leu
1               5                   10

Lys Lys Tyr Phe Asn Xba Gly His Ser Asp Val Ala
        15                  20

Asp Asn Gly Thr Leu Phe Leu Gly Ile Leu Lys Asn
25                  30                  35

Trp Lys Glu Glu Ser Asp Arg Lys Ile Met Gln Ser
            40                  45

Xda Ile Val Ser Phe Tyr Phe Lys Leu Phe Lys Asn
    50                  55                  60

Phe Lys Asp Asp Gln Ser Ile Gln Lys Xab Val Glu
                65                  70

Thr Ile Lys Glu Asp Met Asn Val Xca Phe Phe Asn
        75                  80

Ser Asn Lys Lys Lys Arg Asp Asp Phe Glu Lys Xdb
85                  90                  95

Thr Asn Tyr Ser Val Thr Asp Leu Asn Val Gln Arg
            100                 105

Lys Ala Ile Xbb Glu Leu Ile Gln Val Met Ala Glu
    110                 115                 120

Xcb Ser Pro Ala Ala Lys Thr Gly Lys Arg Lys Arg
                125                 130

Ser Gln Met Leu Phe Arg Gly Arg Arg Ala Ser Gln
        135                 140                 144
 dans laquelle Xaa et Xab, Xba et Xbb, Xca et Xcb ou Xda et Xdb désignent à chaque fois des paires d'aminoacides et les aminoacides d'au moins une de ces paires sont par paires de la cystéine et dans laquelle on a pour les aminoacides qui ne sont pas de la cysteine :
Xaa = Glu, Xab = Ser
Xba = Ala, Xbb = His
Xca = Lys, Xcb = Leu
Xda = Gln, Xdb = Leu,
leurs variantes en terminaisons C raccourcies de 1 à 10 aminoacides et leurs homodimères.

2. Séquence d'ADN
**caractérisée en ce qu'**
elle code pour un polypeptide selon la revendication 1.

3. Vecteur recombinant
**caractérisé en ce qu'**
il contient de l'ADN qui code pour un polypeptide selon la revendication 1.

4. Vecteur recombinant selon la revendication 3,
**caractérisé en ce qu'**
il est un plasmide.

5. Vecteur recombinant selon la revendication 4,
**caractérisé en ce qu'**
il consiste en le plasmide pKK233-2/IFN-γ, dans lequel sa séquence de gène code pour un polypeptide selon la revendication 1.

6. Vecteur recombinant selon la revendication 3,
**caractérisé en ce qu'**
il consiste en un bactériophage qui contient de l'ADN qui code pour un polypeptide selon la revendication 1.

7. Vecteur recombinant selon la revendication 6,
**caractérisé en ce qu'**
il consiste en le bactériophage M13mp19, dans lequel on a clonê une séquence de gène qui code pour un polypeptide selon la revendication 1.

8. Micro-organisme recombinant,
**caractérisé en ce qu'**
il a été transformé avec un vecteur selon l'une quelconque des revendications 3 à 7.

9. Micro-organisme selon la revendication 8,
**caractérisé en ce qu'**
il est une bactérie.

10. Micro-organisme selon la revendication 9,
**caractérisé en ce qu'**
la bactérie est Escherichia coli K12 souche JM105.

11. Procédé de production d'un bactériophage recombinant selon l'une quelconque des revendications 6 ou 7,
**caractérisé en ce que**
le plasmide pKK233-2/IFN-γ est mis à digérer avec des endonucléases de restriction et ensuite le segment d'ADN qui contient la séquence codant pour l'interféron-γ, est cloné d'une manière par ailleurs connue dans l'ADN du bactériophage M13mp18, et on modifie ensuite l'ADN recombinant du bactériophage à l'aide d'oligonucléosides d'une manière également connue par ailleurs, de sorte qu'il code pour un polypeptide selon la revendication 1.

12. Procédé selon la revendication 11,
**caractérisé en ce qu'**
on utilise les endonucléases de restriction EcoRI et HindIII.

13. Procédé de production d'une bactérie recombinante selon l'une quelconque des revendications 6 ou 7,
**caractérisé en ce que**
pour la transfection du micro-organisme on utilise un plasmide selon l'une quelconque des revendications 4 ou 5.

14. Procédé de production d'un polypeptide selon la revendication 1,
**caractérisé en ce qu'**
on cultive une bactérie transformée selon l'une quelconque des revendications 8 à 10, dans une culture on décompose ces cellules et on sépare la protéine d'une manière par ailleurs connue, et on purifie.

15. Procédé selon la revendication 14,
**caractérisé en ce que**
les cellules bactériennes sont décomposées mécaniquement, par exemple par ultrasons.

16. Procédé selon l'une quelconque des revendications 14 ou 15,
**caractérisé en ce que**
la séparation de la protéine s'effectue selon un procédé dans lequel les vésicules cellulaires qui renferment la protéine, sont lavées au moins une fois et on met en solution la protéine ensuite avec du chlorhydrate de guanidine et après séparation on la renature par dilution.

17. Procédé selon l'une quelconque des revendications 14 à 16,
**caractérisé en ce qu'**
on isole la protéine par chromatographie d'affinité sur une colonne ou dans un procédé par lots et ensuite on purifie hautement par filtration sur gel.

18. Procédé selon l'une quelconque des revendications 14 à 17,
**caractérisé en ce que**
dans le procédé par lots le matériau de la chromatographie d'affinité est mis en contact d'une manière régulière avec la solution de protéine afin d'obtenir un dépôt régulier de la résine, et ensuite on lave dans le lot et on élue.

19. Procédé selon l'une quelconque des revendications 17 ou 18,
**caractérisé en ce qu'**
on utilise les matériaux usuels pour la chromatographie d'affinité comme par exemple la sépharose SP ou l'Affi-Gel-Blue.

20. Utilisation d'un polypeptide selon la revendication 1,
en vue de la préparation d'un médicament pour les tumeurs rénales, la granulomatose chronique ou les neurodermites.

21. Utilisation d'un polypeptide selon la revendication 1,
comme produit chimique fin pour des études « in vitro » pour mesurer le taux d'interféron et/ou pour la stimulation des cultures de cellules.
